# EUROPÄISCHE PATENTSCHRIFT

(11) **EP 0 758 637 B1**
(45) Veröffentlichungstag und Bekanntmachung des Hinweises auf die Patenterteilung: **03.11.1999**
(21) Anmeldenummer: 96112425.2
(22) Anmeldetag: 01.08.1996
(51) Int. Cl.: C07C 37/74, C07C 39/16

(54) **Verfahren zur Reinigung von Bisphenol-A**
Process for the purification of bisphenol-A
Procédé de purification du bisphénol-A

(30) Priorität: 14.08.1995 DE 19529855
(43) Veröffentlichungstag der Anmeldung: 19.02.1997
(73) Patentinhaber: BAYER AG, 51368 Leverkusen (DE)
(72) Erfinder: Meurer, Kurt-Peter, Dr., 51375 Leverkusen (DE); Van Osselaer, Tony, Dr., 9100 Belsele (BE); Verhoeven, Werner, Dr., 2920 Heide-Kalmthout (BE); Vaes, Johan, 2920 Kalmthout (BE); Hooftman, Ignace, 9150 Kruibeke (BE); Van Herck, Willy, 2930 Brasschaat (BE); Wulff, Claus, Dr., 47800 Krefeld (DE); Hinz, Jürgen, Dr., 47800 Krefeld (DE); Eitel, Alfred, Dr., 41539 Dormagen (DE); Hallenberger, Kaspar, 51375 Leverkusen (DE)

(56) Entgegenhaltungen:
- EP-A- 0 552 518
- EP-A- 0 679 626
- BE-A- 678 415
- DE-A- 2 918 990

## Beschreibung

Die Erfindung betrifft ein Verfahren zur Herstellung von Bisphenol A, das eine Reinheit von mindestens 99,95 Gew.-% p,p-Bisphenol A (BPA) besitzt. Erfindungsgemäß gereinigtes, thermostabiles und farbstabiles BPA bewirkt u.a. eine verbesserte Thermofarbstabilität und eine verbesserte optische Transparenz in daraus hergestellten Polymeren wie z.B. Polycarbonat.

p,p-Bisphenol-A (BPA) wird z.B. nach bekannten Verfahren aus Aceton und Phenol in Gegenwart von gegebenenfalls modifizierten Ionenaustauscherharzen hergestellt (z.B. DE-A-3 727 641, entsprechend US-A-4 912 263).

Die Herstellung von hochreinem Bisphenol A ist bekannt. In der DE-A-4 213 872 wird beschrieben, wie Bisphenol-A auf einen Reinheitsgrad von 99,91 Gew.-% gebracht werden kann. Die Reinigung erfolgt durch BPA-/Phenol-Adduktkristallisation, anschließende Filtration der Addukte und Desorption des Phenols.

DE-OS 29 18 990 offenbart ein Verfahren zur Herstellung von Bisphenol A, bei dem aus der durch Umsetzung von Phenol und Aceton unter HCl-Katalyse erhaltenen Reaktionsmischung durch Destillation zunächst Wasser und Säure, und anschließend Phenol entfernt werden und das rohe Bisphenol A unter Zusatz eines Stabilisators destillativ gereinigt und anschließend Kristallisiert wird.

Aus EP-A 552 518 geht ein Verfahren zur Gewinnung von Bisphenol A aus der nach Kristallisation eines 1:1 Addukts von Bisphenol A und Phenol verbleibenden Mutterlauge hervor, bei dem nach Isomerisierung und Neutralisation aus der Mutterlauge nacheinander Phenol, gefärbte Nebenprodukte und Bisphenol A destllativ entfernt werden.

In der DE-A-44 13 396 (EP-A 679 626) wird beschrieben, daß man hochreines Bisphenol A erhält, indem man eine Schmelze, die mindestens 90 Gew.-% BPA enthält, zweistufig destilliert und Schwer-/Leicht- und Nicht-Sieder abtrennt. Die verwendete Schmelze wird zunächst durch einen Stickstoffstrom von Phenol befreit und inertisiert. Die Vorreinigung der verwendeten BPA-Schmelze erfolgt durch Kristallisation und Filtration der p,p-Bisphenol-A/Phenol-Mischaddukte.

Es wurde nun gefunden, daß man auf die großtechnisch betriebenen Reinigungsschritte der phenolischen BPA-Lösung wie Kristallisation, Filtration und Desorption der BPA-/Phenol-Addukte verzichten kann, wenn die Reinigung der p,p-Bisphenol enthaltenden, gegebenenfalls inertisierten phenolischen Lösung, wie sie bei der Herstellung von BPA aus Phenol und Aceton anfällt, durch hintereinandergeschaltete Destillationssequenzen erfolgt.

Gegenstand der Erfindung ist daher ein Verfahren zur Gewinnung von Bisphenol A mit einem Reinheitsgrad >99,95 Gew.-% und einer Hazen-Farbzahl (DIN 53 409) <5 aus der Reaktionslösung, die bei der Herstellung von BPA aus Aceton und Phenol in Gegenwart von gegebenenfalls mit Mercaptoaminen und/oder Thiazolidinen und/oder Thiocarbonsäuren modifizierten sulfonsauren Ionenaustauschern erhalten wird, dadurch gekennzeichnet, daß
a) die phenolische Lösung bei einer Temperatur von 60 bis 80°C auf einen Gehalt an p,p-BPA von 10 bis 40 Gew.-% eingestellt wird,
b) anschließend die so hergestellte gegebenenfalls inertisierte Lösung einer Sequenz hintereinandergeschalteter Kolonnen zugeführt wird, wobei
c) in einem ersten Destillationsschritt die Entfernung von Wasser und Restaceton, gegebenenfalls Phenol, aus der aus dem Reaktor ausgetretenen Reaktionslösung erfolgt, dann
d) in einem zweiten Destillationsschritt die so hergestellte, entwässerte Lösung im Sumpf der Kolonne auf 70 bis 97 Gew.-% p,p-BPA-Gehalt eingestellt wird, wobei am Kopf der Kolonne Phenol entfernt wird, dann
e) in einem dritten Destillationsschritt die so erhaltene Lösung von Schwer- und Nichtsieder-Bestandteilen gereinigt wird, dann
f) in einem vierten Destillationsschritt die mit dem p,p-BPA überdestillierten Leichtsieder und Restphenol abgetrennt werden, dann
g) die ex Sumpf erhaltene p,p-BPA-Schmelze auf übliche Weise aufgearbeitet wird.

Das auf diese Weise hergestellte p,p-Bisphenol A wird als Rohstoff für Polymere wie Polycarbonate oder Epoxy-Harze eingesetzt.

Daß die Reinigung der phenollschen BPA-Lösung lediglich durch sequentiell hintereinandergeschaltete Destillationsschritte bewerkstelligt werden kann, war nicht vorhersehbar, da bekanntlich BPA und die enthaltenden Isomeren bei sehr hohen Temperaturen sich zersetzen bzw. isomerisieren und daher eine schlechte Farbzahl der BPA-Schmelze und der BPA-Feststoffware resultiert.

Das Destillationsverfahren der phenolischen BPA-Lösung hat zudem den Vorteil, daß man auf aufwendige mehrstufige Kristallisationsverfahren, den Einsatz von Drehfiltern und Zentrifugen sowie die Verwendung störanfälliger Desorptionsaggregate verzichten kann. Somit wird der technisch aufwendige, wartungsintensive Betrieb von Prozeßapparaten vermieden und damit die Verfügbarkeit der Anlage erhöht.

### Beispiele

### Beispiel 1 Herstellung Reaktionslösung

In einem doppelwandigen Glasreaktor (10 l), der auf 80°C thermostatisiert wird, werden über Glaswollepackungen 8 l phenolfeuchtes sulfoniertes Polystyrolharz (Lewatit SC 102, Bayer AG) vorgelegt. Es wird kontinuierlich mit Stickstoff inertisiert. Dann wird kontinuierlich eine Lösung (1 l/h) von 95,5 % Phenol und 4,5 % Aceton, die auf 70°C vorgewärmt wurde, von oben in den Reaktor eingespeist.

Am unteren Ablauf des Reaktors erhält man eine phenolische Lösung folgender Zusammensetzung: Phenol: 84 %; Aceton: 1 %; Wasser: 1,2 %; p,p-BPA: 11,6 %; o,p-BPA: 1,6 %; DMX: 24 ppm; Chromane: 3 700 ppm; Indane: 42 ppm; Trisophenol: 1 780 ppm; Mol 402: 410 ppm; Rest: 110 ppm.

### Beispiel 2 Destillation Aceton/Wasser aus Reaktionslösung

In einem Dreihalskolben werden 10 l einer phenolischen Lösung obiger Konsistenz (Ablauf Reaktor) vorgelegt und über zwei Plattenkolonnen mit 10 Trennstufen inertisiert destilliert. Die Destillation erfolgt bei 150 mbar, die Bodentemperatur beträgt 126°C.

Man erhält ein Destillat mit folgender Zusammensetzung: Aceton: 37,5 %; Wasser: 58,5 %; Phenol: 4 %, Mesitylen: 25 ppm; Anisol: 10 ppm; Methanol: 300 ppm.

Das Bodenprodukt setzt sich wie folgt zusammen: Phenol: 85,9 %; p,p-BPA: 11,9 %; o,p-BPA: 1,6 %; DMX: 25 ppm; Chromane: 3 765 ppm; Indane: 43 ppm; Trisphenol: 1 820 ppm; Mol 402: 420 ppm; Rest: 102 ppm.

### Beispiel 3 Phenoldestillation der entwässerten Lösung

Die Phenoldestillation erfolgte bei 100 mbar, die Kopftemperatur betrug 120°C. Das Destillat enthält 99,95 % Phenol und 50 ppm o-Kresol. Das Bodenprodukt enthält: 4 400 ppm Phenol; 83,7 % p,p-BPA; 11,4 % o,p-BPA; 173 ppm DMX; 2,7 % Chromane; 303 ppm Indane; 1,3 % Trisphenol; 3 000 ppm Mol 402 und 700 ppm Restisomeren.

### Beispiel 4 Abtrennung Schwersieder/Leichtsieder

Die obige Lösung wird bei 2 mbar und einer Bodentemperatur von 226°C destilliert: Zunächst werden Schwersieder in der Kolonne im Sumpf angereichert und ausgeschleust.

Die Leichtsieder und BPA werden über Kopf destilliert und in eine 2. Kolonne geführt; dort werden die Leichtsieder und Restphenol bei 2 mbar über Kopf abgetrennt. Das Sumpfprodukt enthält hochreines p,p-BPA (>99,95 Gew.-%).

Diese Destillation erfolgt z.B. nach einem bekannten Verfahren zur Destillation von BPA-Schmelze, das in der DE-A-44 13 396 beschrieben ist. Man erhält ein Bisphenol A (in 98 %iger Ausbeute) mit einer Reinheit von 99,98 Gew.-% p,p-BPA.

In der Bilanz beträgt der Verlust an p,p-BPA <2 %.

## Patentansprüche

1. Verfahren zur Gewinnung von Bisphenol A mit einem Reinheitsgrad >99,95 Gew.-% und einer Farbzahl von Hazen <5 aus der Reaktionslösung, die bei der Herstellung von Bisphenol A aus Aceton und Phenol in Gegenwart von gegebenenfalls mit Mercaptoaminen und/oder Thiazolidinen und/oder Thiocarbonsäuren modifizierten sulfonsauren Ionenaustauschern erhalten wird, dadurch gekennzeichnet, daß
a) die phenolische Lösung bei einer Temperatur von 60 bis 80°C auf einen Gehalt an p,p-BPA von 10 bis 40 Gew.-% eingestellt wird,
b) anschließend die so hergestellte gegebenenfalls inertisierte Lösung einer Sequenz hintereinandergeschalteter Kolonnen zugeführt wird, wobei
c) in einem ersten Destillationsschritt die Entfernung von Wasser und Restaceton, gegebenenfalls Phenol, aus der aus dem Reaktor ausgetretenen Reaktionslösung erfolgt, dann
d) in einem zweiten Destillationsschritt die so hergestellte, entwässerte Lösung im Sumpf der Kolonne auf 70 bis 97 Gew.-% p,p-BPA-Gehalt eingestellt wird, wobei am Kopf der Kolonne Phenol entfernt wird, dann
e) in einem dritten Destillationsschritt die so erhaltene Lösung von Schwer- und Nichtsieder-Bestandteilen gereinigt wird dann
f) in einem vierten Destillationsschritt die mit dem p,p-BPA überdestillierten Leichtsieder und Restphenol abgetrennt werden, dann
g) die ex Sumpf erhaltene p,p-BPA-Schmelze auf übliche Weise aufgearbeitet wird.

## Claims

1. Process for the recovery of bisphenol A, having a degree of purity of >99.95 wt.% and a Hazen colour index of <5, from the reaction solution obtained in the preparation of bisphenol A from acetone and phenol in the presence of sulfonic acid ion exchangers optionally modified with mercaptoamines and/or thiazolidines and/or thiocarboxylic acids,
characterised in that
a) the phenolic solution, at a temperature of from 60 to 80°C, is adjusted to a p,p-BPA content of from 10 to 40 wt.%,
b) the optionally inertised solution thus prepared is then passed to a sequence of columns connected in tandem, wherein
c) in a first distillation step, water and residual acetone and possibly phenol are removed from the reaction solution issuing from the reactor, then
d) in a second distillation step, the dehydrated solution thus obtained in the bottom of the column is adjusted to a p,p-BPA content of from 70 to 97 wt.%, phenol being removed at the top of the column, then
e) in a third distillation step, the solution of high-boiling and non-boiling components thus obtained is purified, then
f) in a fourth distillation step, the low-boiling components and residual phenol which have distilled over with the p,p-BPA are separated off, then
g) the p,p-BPA melt obtained from the bottom of the column is worked up in the conventional manner.

## Revendications

1. Procédé pour l'obtention de Bisphénol A possédant un degré de pureté > 99,95% en poids et un indice de trouble de Hazen < 5 à partir de la solution réactionnelle que l'on obtient lors de la préparation du Bisphénol A à partir d'acétone et de phénol en présence d'échangeurs d'ions sulfonés, le cas échéant modifiés avec des mercaptoamines et/ou avec des thiazolidines et/ou avec des acides thiocarboxyliques,
caractérisé en ce que
a) on règle la teneur en p,p-BPA de la solution phénolique à une température de 60 à 80°C à une valeur de 10 à 40% en poids,
b) on achemine ensuite la solution ainsi préparée, le cas échéant rendue inerte à une séquence de colonnes montées les unes derrière les autres, dans lesquelles
c) dans une première étape de distillation, on élimine l'eau et l'acétone résiduelle, le cas échéant le phénol de la solution réactionnelle évacuée du réacteur, puis
d) dans une deuxième étape de distillation, on règle dans le produit de bas de colonne la teneur en p,p-BPA de la solution déshydratée ainsi obtenue, à une valeur de 70 à 97% en poids, le phénol étant éliminé à la tête de la colonne, puis
e) dans une troisième étape de distillation, on purifie la solution ainsi obtenue des constituants entrant difficilement en ébullition et des constituants n'entrant pas en ébullition, ensuite
f) dans une quatrième étape de distillation, on sépare les produits entrant aisément en ébullition et le phénol résiduel distillés avec le p,p-BPA, ensuite
g) on traite de la manière habituelle la masse fondue de p,p-BPA obtenue à partir du produit de bas de colonne.
